# EUROPÄISCHE PATENTSCHRIFT

(11) **EP 1 389 471 B1**
(45) Veröffentlichungstag und Bekanntmachung des Hinweises auf die Patenterteilung: **16.08.2006**
(21) Anmeldenummer: 03090188.8
(22) Anmeldetag: 21.06.2003
(51) Int. Cl.: A61L 31/10

(54) **Verfahren zur Herstellung eines implantationsfähigen Stents mit einer polymeren Beschichtung aus hochmolekularem Poly-L-Lactid**
Method for producing an implantable stent with a polymeric coating of high-molecular poly-L-lactide
Procédé de préparation d'un stent implantable ayant un revêtement de poly-L-lactide de poids moléculaire élevé

(30) Priorität: 13.08.2002 DE 10237572
(43) Veröffentlichungstag der Anmeldung: 18.02.2004
(73) Patentinhaber: BIOTRONIK GmbH & Co. KG, 12359 Berlin (DE)
(72) Erfinder: Heublein, Bernd, 30627 Hannover (DE); Sternberg, Katrin, 18057 Rostock (DE); Tittelbach, Michael, 90429 Nürnberg (DE)
(74) Vertreter: Lindner-Vogt, Karin L.

(56) Entgegenhaltungen:
- LINCOFF A M ET AL: "Sustained local delivery of dexamethasone by a novel intravascular eluting stent to prevent restenosis in the porcine coronary injury model" JOURNAL OF THE AMERICAN COLLEGE OF CARDIOLOGY 1997 UNITED STATES, Bd. 29, Nr. 4, 1997, Seiten 808-816, XP002261857 ISSN: 0735-1097
- AGRAWAL C.M. ET AL.: "Evaluation of poly(L-lactic acid as a material for intravascular polymeric stents" BIOMATERIALS, Bd. 13, Nr. 3, 1992, Seiten 176-182, XP002261858

## Beschreibung

Die Erfindung betrifft Verfahren zur Herstellung eines implantationsfähigen Stents mit einer polymeren Beschichtung aus hochmolekularen Poly-L-lactid.

Einer der häufigsten Todesursachen in Westeuropa und Nordamerika sind koronare Herzkrankheiten. Nach neuen Erkenntnissen sind vor allem entzündliche Prozesse treibende Kraft der Arteriosklerose. Initiiert wird der Prozess vermutlich durch die vermehrte Einlagerung von Lipoproteinen geringer Dichte (LDL-Partikel) in die Intima der Gefäßwand. Nach dem Eindringen in die Intima werden die LDL-Partikel durch Oxidantien chemisch modifiziert. Die modifizierten LDL-Partikel veranlassen wiederum die Endothelzellen, die die inneren Gefäßwände auskleiden, das Immunsystem zu aktivieren. In der Folge treten Monocyten in die Intima ein und reifen zu Makrophagen heran. Im Zusammenspiel mit den ebenfalls eintretenden T-Zellen werden Entzündungsmediatoren, wie Immunbotenstoffe und proliferativ wirkende Substanzen, freigesetzt, und die Makrophagen beginnen die modifizierten LDL-Partikel aufzunehmen. Die sich bildenden Lipidläsionen aus T-Zellen und den mit LDL-Partikeln gefüllten Makrophagen, die aufgrund ihres Aussehens Schaumzellen genannt werden, stellen eine Frühform der arteriosklerotischen Plaque dar. Die Entzündungsreaktion in der Intima veranlasst durch entsprechende Entzündungsmediatoren glatte Muskelzellen der weiter außen liegenden Media der Gefäßwand bis unter die Endothelzellen zu wandern. Dort vermehren sie sich und bilden eine fibröse Deckschicht aus dem Faserprotein Kollagen, die den darunter liegenden Lipidkern aus Schaumzellen gegen die Blutbahn abgrenzt. Die dann vorliegenden tiefgreifenden strukturellen Veränderungen in der Gefäßwand werden zusammenfassend als Plaque bezeichnet.

Die arteriosklerotische Plaque expandiert zunächst relativ wenig in Richtung der Blutbahn, da diese sich kompensatorisch erweitern kann. Mit der Zeit kommt es jedoch zu einer Verengung des Blutkanals (Stenose), deren erste Anzeichen bei körperlicher Belastung auftreten. Die verengte Arterie kann sich dann nicht mehr ausreichend weiten, um das zu versorgenden Gewebe besser zu durchbluten. Ist eine Herzarterie betroffen, so klagt der Patient häufig über ein Druck- und Engegefühl hinter dem Brustbein (Angina pectoris). Bei anderen Arterien sind schmerzhafte Krämpfe ein häufig auftretendes Indiz für die Stenose.

Die Stenose kann letztendlich zu einem völligen Verschließen der Blutbahn führen (Herzinfarkt, Schlaganfall). Allein durch die Plaquebildung tritt dies nach neueren Untersuchungen jedoch nur in etwa 15 Prozent der Fälle auf. Vielmehr scheint der durch bestimmte Entzündungsmediatoren aus den Schaumzellen veranlasste allmähliche Abbau der fibrösen Deckschicht aus Kollagen ein entscheidender Zusatzfaktor zu sein. Reißt die fibröse Deckschicht auf, so kann der Lipidkern unmittelbar mit dem Blut in Kontakt kommen. Da in Folge der Entzündungsreaktion gleichzeitig Gewebsfaktoren (TF tissue factor) in den Schaumzellen produziert werden, die sehr potente Auslöser der Gerinnungskaskade sind, kann der sich bildende Blutpfropf das Blutgefäß verschließen.

Seit mehr als zwanzig Jahren sind nicht-operative Methoden zur Stenose-Behandlung etabliert, bei denen u.a. durch Ballondilatation (PTCA Perkutane Transluminale Coronare Angioplastie) das Blutgefäß wieder aufgeweitet wird. Mit dem Aufweiten des Blutgefäßes entstehen allerdings überwiegend Verletzungen, Einrisse, Disselektionen in der Gefäßwand, die zwar problemlos verheilen, jedoch in etwa einem Drittel der Fälle durch das ausgelöste Zellwachstum zu Wucherungen führen (Proliferation), die letztendlich zu einer erneuten Gefäßverengung (Restenose) führen. Die Aufweitung beseitigt auch nicht die physiologischen Ursachen der Stenose, also die Veränderungen in der Gefäßwand. Eine weitere Ursache der Restenose ist die Elastizität des gedehnten Blutgefäßes. Nach dem Entfernen des Ballons zieht sich das Blutgefäß übermäßig zusammen, so dass der Gefäßquerschnitt verringert wird (Obstruktion, sogenanntes negatives remodelling). Letzterer Effekt kann nur durch Platzierung eines Stents vermieden werden. Durch den Einsatz von Stents kann zwar ein optimaler Gefäßquerschnitt erreicht werden, allerdings führt der Einsatz von Stents ebenfalls zu kleinsten Verletzungen, die die Proliferation induzieren können und damit letztendlich eine Restenose auslösen können.

Mittlerweile bestehen umfangreiche Erkenntnisse zum zellbiologischen Mechanismus und zu den auslösenden Faktoren der Stenose und Restenose. Die Restenose entsteht - wie bereits erläutert - als Reaktion der Gefäßwand auf die lokale Verletzung infolge Dehnung der artherosklerotischen Plaque. Über komplexe Wirkmechanismen wird die lumengerichtete Migration und Proliferation der glatten Muskelzellen der Media und der Adventitia induziert (neointimale Hyperplasie). Unter Einfluss verschiedener Wachstumsfaktoren produzieren die glatten Muskelzellen eine Deckschicht aus Matrixproteinen (Elastin, Kollagen, Proteoglykane), deren ungesteuertes Wachstum allmählich zu einer Einengung des Lumens führen kann. Systematisch medikamentöse Therapieeinsätze sehen u.a. die orale Verabreichung von Calcium-Antagonisten, ACE-Hemmern, Antikoagulantien, Antiaggregantien, Fischölen, antiproliferativen Substanzen, antiinflammatorischen Substanzen und Serotonin-Antagonisten vor, signifikante Reduktionen der Restenoseraten wurden auf diesem Wege bisher jedoch nicht erreicht.

Seit einigen Jahren versucht man die Restenosegefahr bei der Implantation von Stents durch Aufbringung spezieller Beschichtungen zu mindern. Teilweise dienen die Beschichtungssysteme selbst als Trägermatrix, in die ein oder mehrere Arzneistoffe eingebettet sind (Local Drug Delivery). Die Beschichtung bedeckt in der Regel zumindest eine der Gefäßwand zugewandte Oberfläche des endovaskulären Implantates.

Die Beschichtungen bestehen nahezu zwangsläufig aus einem biokompatiblen Material, welches entweder natürlichen Ursprungs ist oder auf synthetischem Wege gewonnen werden kann. Eine besonders gute Verträglichkeit und die Möglichkeit die Elutionscharakteristik des eingebetteten Arzneistoffs zu beeinflussen bieten biodegradierbare Beschichtungsmaterialien. Beispiele für die Verwendung biodegradierbarer Polymere sind Cellulose, Kollagen, Albumin, Casein, Polysaccharide (PSAC), Polylactid (PLA) , Poly-L-lactid (PLLA), Polyglykol (PGA), Poly-D,L-lactid-co-glycolid (PDLLA/PGA), Polyhydroxybuttersäure (PHB), Polyhydroxyvaleriansäure (PHV), Polyalkylcarbonate, Polyorthoester, Polyethylenterephthalat (PET), Polymalonsäure (PML), Polyanhydride, Polyphosphazene, Polyaminosäuren und deren Copolymere sowie Hyaluronsäure und seine Derivate.

Zahlreiche Studien belegen mittlerweile den positiven Effekt von biokompatiblen Beschichtungen auf die Restenoseneigung bei metallischen Stents. Trotz dieser Erfolge verbleibt auch bei den bisher eingesetzten Materialien noch ein nicht unerhebliches Restrisiko zur Restenosebildung. Gerade die besonders kostengünstige, leicht verarbeitbaren und als Polymermatrix für die Aufnahme von Arzneimitteln besonders geeigneten Polylactide zeigen bei Verwendung von Chargen herkömmlicher Qualität und Molekulargewichtes einen nachteiligen inflammatorischen Reiz auf die Gewebsumgebung.

Für die meisten technischen Anwendungen werden Polylactide mit Molmassen im Bereich von etwa 60 bis 200 kDa eingesetzt (siehe beispielsweise H. Saechtling; Kunststoff Taschenbuch; 28. Ausgabe; S.611). Dementsprechend wurden auch die bisher im medizinischen Gebiet der Implantationstechnik verwendeten Polylactide aus diesem Molmassenbereichen ausgewählt.

Zur Minderung der thrombogenen Eigenschaften von Stents wurde ein Polylactid (PDLLA) beschichteter Stent mit einem eingebetteten Thrombininhibitor vorgeschlagen (Hermann R., Schmidmaier G., Märkl B., Resch A., Hähnel I., Stemberger A., Alt E.; Antithrombogenic Coating of Stents Using a Biodegradable Drug Delivery Technology; Thrombosis and haemostasis, 82 (1999) 51-57). Die verwendete Polymermatrix aus PDLLA hatte ein durchschnittliches Molekulargewicht von etwa 30 kDa. Eine etwa 10 µm dicke Beschichtung desselben polymeren Werkstoffs diente einer anderen Studie zufolge als Träger der Wirkstoffe Hirudin und lloprost (Alt E., Hähnel I. et al.; Inhibition of Neointima Formation After Experimental Coronary Artery Stenting; Circulation, 101 (2000) 1453-1458).

Einer weiteren Studie zufolge wurde u.a. PLLA mit einer Molmasse von etwa 321 kDa zur Beschichtung eines Koronarstents verwendet. In weiteren Untersuchungen wurde als Wirkstoff Dexamethason der Polymermatrix beigesetzt. Eine Sterilisation erfolgte nach konventioneller Ethylenoxid-Technik. Die beschichteten Stents wurden in Schweine implantiert und nach 28 Tagen erfolgte eine histologische Analyse der neointimalen Hyperplasie (Lincoff A.M., Furst J.G., Ellis S.G., Tuch R.J., Topol E.J.; Sustained Local Delivery of Dexamethasone by a Novel Intravascular Eluting Stent to Prevent Restenosis in the Porcine Coronary Injury Model; Journal of the American College of Cardiology, 29 (1997), 808-816).

In der DE 198 43 254 ist der Einsatz eines Blends aus Poly-L-lactid (Charge L104 der Firma Boehringer Ingelheim) und Polycyanacrylsäureester oder Polymethylenmalonsäureester als Beschichtungsmaterial für Implantate beschrieben. Die angegebene Charge besitzt nach Herstellerangaben ein durchschnittliches Molekulargewicht von etwa 2 kDa. Auch aus der US 6,319,512 ist ein Implantat zur Wirkstoffabgabe bekannt, dessen Hülle aus einem Blend von Poly-L-lactid der Charge L104 und einem Copolymer aus Lactid und Glykol besteht.

Mitunter soll die inflammatorische Wirkung von Poly-L-lactid ausgenutzt werden, um die Gewebsneubildung anzuregen. So wird in der US 2002/0040239 vorgeschlagen bei schlecht abheilenden Gewebsverletzungen kleine Implantate aus u.a. Poly-L-lactid in das Gewebe einzubringen. Angaben zur Molmasse des Polymers werden nicht gemacht, so dass offensichtlich davon ausgegangen wird, dass ein Poly-L-lactid mit üblicher Zusammensetzung zur Erzeugung des Effektes ausreicht.

Ferner ist der Einsatz von Poly-L-lactiden als Werkstoff für Stents beschrieben wurden. So in der EP 0 574 474 in amorph / kristallinen Polymergemischen mit Weichmacher, in der US 6,368,346 als Bestandteil eines Blends und in klinischen Studien am Menschen (Tsuji T., Tamai H., Igaki K. et al.; One year follow-up of biodegradable self-expanding stent implantation in humans; Journal of the American College of Cardiology, 37 (2001), 47A). Allerdings sind die mechanischen Eigenschaften von Stents aus Polymeren, insbesondere auf Basis biodegradierbarer Polymere, gegenüber metallischen Stents deutlich schlechter. Die hohe Biegesteifigkeit, das bessere Recoil-Verhalten, die bessere Bruchdehnung und die einfachere Verarbeitung sprechen derzeit für metallische Stents mit einer polymere Beschichtung anstelle eines Implantats aus Vollkunststoff. Wird der Werkstoff Poly-L-lactid als Volumenmaterial zur Herstellung von Stents eingesetzt, so führen die bekannten Verarbeitungstechniken (Coextrusion, Spritzgusstechnik, etc.) zu sehr spezifischen Änderungen der Werkstoffeigenschaften, z.B. einem Anstieg der Dichte und Steifigkeit und einer Abnahme der Porosität. Wird dagegen das Polymer als Beschichtungsmaterial aufgebracht, so sind nicht nur andere Werkstoffeigenschaften gewünscht, sondern resultieren bereits aus dem stark unterschiedlichen Herstellungsweg (z.B. Sprüh- oder Tauchverfahren). Daher erlaubt die Verwendung eines Polymers als Volumenmaterial keinen Rückschluss auf die Eigenschaften desselben Materials als Beschichtung.

Für die Anwendung am Menschen ist es unerlässlich den Stent zu Sterilisieren. Zur Herstellung eines implantationsfähigen Stents muss demnach der polymeren Beschichtung immer eine Sterilisation folgen. Gängige Sterilisationsverfahren für Polylactide, insbesondere die aus dem Stand der Technik bekannten zugelassenen Verfahren Wasserdampfsterilisation, Plasmasterilisation mit Wasserstoffperoxid und Ethylenoxid-Sterilisation führen zu einer Reduktion der Molekularmasse des Polymers und einer teils erheblichen Beeinträchtigung der Formstabilität der Beschichtung. Ein Grund dafür dürfte in den jeweils vorhandenen Schritten zur Durchfeuchtung des Sterilisationsgutes liegen, da Polylactide unter Einwirkung von Wasser bzw. Wasserstoffperoxid infolge hydrolytischer Prozesse degradieren. Lange Expositionszeiten bei wasserfreien Verfahren, wie der Gammastrahlensterilisation, führen zu strukturellen Veränderungen im Polymer durch Radikalbildung. Werden arzneistoffbeladene Beschichtungen sterilisiert, dann reduzieren die erwähnten Verfahren zudem die biologische Wirksamkeit der enthaltenen Wirkstoffe.

Aufgabe der vorliegenden Erfindung ist es, ein Verfahren zur Herstellung derartig beschichteter Stents bereitgestellt bereitzustellen, das den besonderen Anforderungen an das Beschichtungsmaterial genügt.

Die Aufgabe wird durch einen Stent mit den im Anspruch 1 genannten Merkmalen das Herstellungsverfahren nach Anspruch 1 gelöst.

Nach dem erfindungsgemäßen Verfahren zur Herstellung eines implantationsfähigen Stents ist vorgesehen, dass der Stent
(a) zumindest abschnittsweise mit einem feinen Nebel aus einer Lösung von Poly-L-lactid des durchschnittlichen Molekulargewichts von mehr als 650 kDa benetzt wird,
(b) die auf dem Stent aufgebrachte Lösung durch Abblasen getrocknet wird und
(c) der Stent anschließend mittels Elektronenstrahlsterilisation mit einer Dosierung im Bereich von 15 bis 35 kGy und einer kinetischen Energie der Elektronen im Bereich von 4 bis 5 MeV sterilisiert wird.

Die Aufbringung der polymeren Beschichtung erfolgt vorzugsweise mit Rotationszerstäubern, die einen fein verteilten Nebel aus kleinsten Schwebeteilchen erzeugen. Aus einem Vorratsbehälter wird dazu eine Lösung des hochmolekularen Polymers, gegebenenfalls mit einem oder mehreren Wirkstoffen versetzt, entnommen. Der feine Sprühnebel benetzt oberflächlich kleinste Strukturen des Implantats und wird anschließend durch Abblasen getrocknet. Dieser Vorgang kann beliebig wiederholt werden, bis die gewünschte Schichtdicke der polymeren Beschichtung erreicht ist. Anschließend erfolgt die Elektronenstrahlsterilisation.

Das Sterilisationsverfahren hat sich als besonders geeignet für Polylactide gegenüber anderen zugelassenen Verfahren erwiesen. Die Elektronenstrahlsterilisation hat keinen oder nur einen geringen Einfluss auf die Formstabilität der polymeren Beschichtung und die biologische Wirksamkeit eines eventuell eingebetteten Wirk stoffs. Die nur wenige Sekunden langen Expositionszeiten bei der Elektronenstrahlsterilisation verhindern unerwünschte strukturelle Veränderungen im Polymer durch Radikalbildung. Durch die Sterilisation muss zwar eine deutliche Reduktion des Molekulargewichtes in Kauf genommen werden, jedoch lässt sich der Vorgang durch Vorgabe entsprechend Parameter steuern. Als besonders praktikabel hat sich in der Praxis eine Bestrahlung mit einer Dosierung im Bereich von 15 bis 35 kGy, insbesondere im Bereich von 22 bis 28 kGy, erwiesen. Eine kinetische Energie der Elektronen liegt erfindungsgemäß im Bereich von 4 bis 5 MeV. Mit sinkender Dosierung und/oder sinkendender kinetischer Energie der Elektronen kann die Reduktion des Molekulargewichtes infolge der Sterilisation verringert werden. Betriebsparameter für die Sterilisation, die zur Einstellung eines konkret gewünschten Molekulargewichtes führen, sind gerätespezifisch zu ermitteln. Weiterhin sind die Betriebsparameter auch für das jeweilige Substrat zu spezifizieren, denn herstellungsbedingte Variationen der Eigenschaften des polymeren Beschichtung, wie z.B. deren Schichtdicke und spezifischen Dichte, haben ebenfalls Einfluss auf den Umfang des Crack-Prozesses. In der Regel verringert sich der Abbau des Molekulargewichtes mit zunehmender Schichtdicke und spezifischer Dichte der Beschichtung.

Dadurch, dass die polymere Beschichtung im implantationsfähigen Zustand nach Herstellung und Sterilisation Poly-L-lactid mit einem durchschnittlichen Molekulargewicht von mehr als 200 kDa, insbesondere von mehr als 350 kDa enthält, lassen sich die Restenose auslösenden Faktoren offensichtlich wirkungsvoll unterdrücken. Überraschenderweise zeigte sich, dass die neointimale Proliferation mit derartig hochpolymeren Beschichtungen deutlich gesenkt werden konnte. Offenbar führt der Einsatz des hochmolekularen Polymers gegenüber kürzerkettigen Polymeren zu einer deutlichen Minderung inflammatorischer und proliferativer Prozesse.

Die im erfindungsgemäßen Sinne verwendeten Angaben zum Molekulargewicht beziehen sich auf nach der Formel von Mark-Houwink (MH) bestimmte Werte. Für das beispielhaft eingesetzte Poly-L-Latid L214 der Fa. Boehringer Ingelheim liegt das Molekulargewicht vor der Sterilisation nach Herstellerangaben bei 691 kDa. Nach der im erfindungsgemäßen Herstellungsverfahren erfolgten Elektronenstrahlsterilisation wurden u.a. Molekulargewichte zwischen 220 kDa und 245 kDa nach demselben Verfahren bestimmt.

Das hochmolekulare Poly-L-lactid eignet sich insbesondere als Arzneistoffträger für pharmakologisch aktive Arzneistoffe. Soll daher ergänzend eine pharmakologische Therapie auf lokaler Ebene durchgeführt werden, so können ein oder mehrere Wirkstoffe in an sich bekannter Weise - zumeist einhergehend mit der Aufbringung der polymeren Beschichtung - eingebettet werden.

Eine Schichtdicke der polymeren Beschichtung liegt sowohl im Falle der Zusatzfunktion als Arzneistoffträger als auch in Alleinanwendung bei vorzugsweise 3 bis 30 µm, insbesondere 8 bis 15 µm. Mit den gewählten Bereichen lässt sich ein ausreichend hoher Benetzungsgrad der Oberfläche des Stents sicherstellen. Derartig dünne Beschichtungen neigen jedoch noch nicht zur Rissbildung und wirken demnach einem Abblättern bei mechanischer Beanspruchung des Stents entgegen. Insgesamt werden pro Stent vorzugsweise 0,3 bis 2 mg, insbesondere 0,5 bis 1 mg, an Beschichtungsmaterial aufgebracht. Um inflammatorische Reaktionen zu unterdrücken, sollte das Implantat möglichst großflächig mit der polymeren Beschichtung bedeckt sein.

Ferner ist es vorteilhaft, wenn ein Grundkörper des Implantates aus zumindest einem Metall oder zumindest einer Metalllegierung geformt ist. Vorteilhaft ist weiterhin, wenn das Metall oder die Metalllegierung zumindest teilweise biodegradierbar ist. Die biodegradierbare Metalllegierung kann insbesondere eine Magnesiumlegierung sein. Der Stent wird nach der biodegradierbaren Variante mit der Zeit vollständig abgebaut und damit verschwinden auch mögliche Auslöser für eine inflammatorische und proliferative Reaktion des umgebenden Gewebes.

Im Falle von wirkstoffbeladenen polymeren Beschichtungen sollte ein Stentdesign vorzugsweise derart angepasst sein, dass ein möglichst großflächiger Kontakt zur Gefäßwand besteht. Dies begünstigt eine gleichmäßige Elution des Wirkstoff, die Untersuchen zufolge im wesentlichen diffusionskontrolliert ist. Bereiche hoher mechanischer Verformbarkeit sind vorzugsweise bei der Beschichtung auszusparen, da hier die Gefahr des Abblätterns der Beschichtung erhöht ist. Alternativ oder in Ergänzung hierzu kann das Stentdesign derart vorgegeben werden, dass bei mechanischer Belastung, d.h. in der Regel bei der Dilatation des Stents, eine möglichst gleichmäßige Verteilung der auftretenden Kräfte über die gesamte Stentoberfläche gegeben ist. Auf diese Weise können lokale Überbeanspruchungen der Beschichtung und damit eine Rissbildung oder gar ein Abblättern der Beschichtung vermieden werden.

Ein sehr hohes Haftungsvermögen zeigt die polymere Beschichtung, wenn das Implantat eine passive Beschichtung aus amorphem Siliziumkarbid aufweist. Die polymere Beschichtung kann direkt auf die passive Beschichtung aufgebracht werden. Alternativ können Spacer oder Haftvermittlerschichten, die auf der passiven Beschichtung angebunden werden, zur weiteren Steigerung des Haftvermögens der polymeren Beschichtung vorgesehen sein.

Die Erfindung wird nachfolgend anhand eines Ausführungsbeispiels und anhand der Zeichnungen näher erläutert. Es zeigen:
- Figur 1: eine schematische Draufsicht auf einen Teilausschnitt eines endovaskulären Implantats in Form eines Stents;
- Figur 2: eine Schnittansicht durch ein Strukturelement des Stents mit einer polymeren Beschichtung und
- Figur 3: ein zur Figur 1 alternatives Stentdesign.

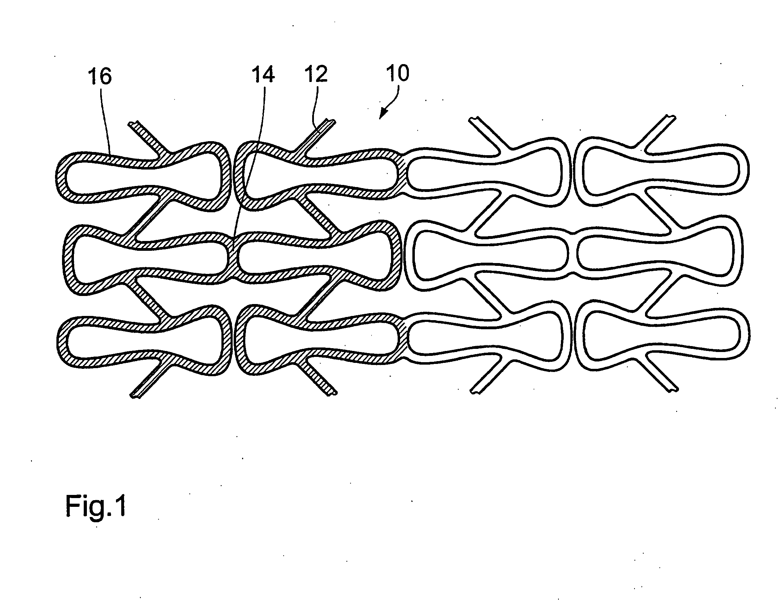

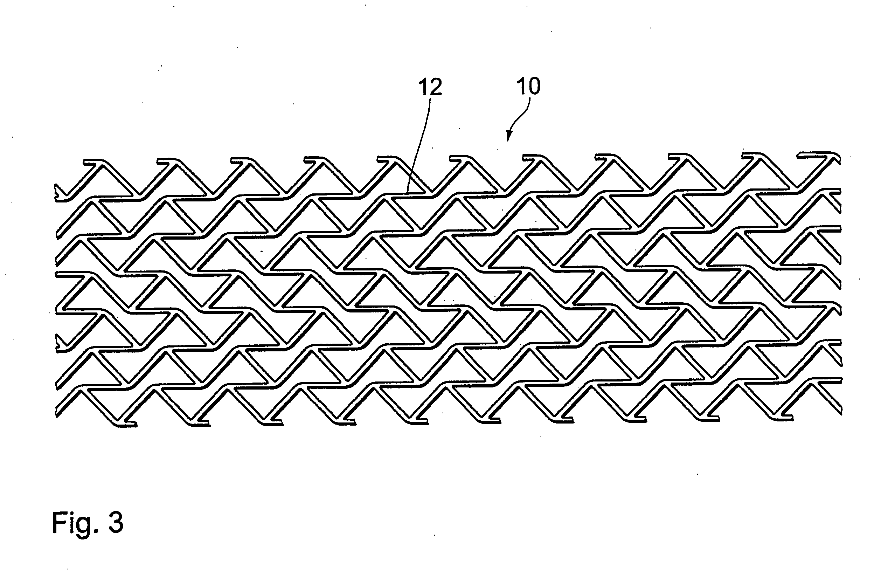

Die Figur 1 zeigt in eine schematische Draufsicht eines Teilausschnitts aus einem endovaskulären Implantat, hier in Form eines Stents 10. Der Stent 10 besteht aus einer Vielzahl von Strukturelementen 12, die - wie in diesem speziellen Beispiel dar gestellt - ein netzartiges Muster um eine Längsachse des Stents 10 bilden. Derartige Stents sind seit langem in der Medizintechnik bekannt und können, was ihren strukturellen Aufbau angeht, im hohen Maße variieren. Von Bedeutung mit Sicht auf die vorliegende Erfindung ist, dass der Stent 10 eine nach außen gewandte, also nach Implantation zur Gefäßwand gerichtete Oberfläche 14 besitzt. Diese sollte im expandierten Zustand des Stents 10 eine möglichst große Flächenabdeckung besitzen, um eine gleichmäßige Wirkstoffabgabe zu ermöglichen. In Hinblick auf die mechanische Grundstruktur sind bei der Formgebung zu unterscheiden: Konzentration der Verformung auf wenige Bereiche oder gleichmäßige Verformung über die gesamte Grundstruktur. Im ersteren Falle sind die Strukturen derart ausgebildet, dass bei einer mechanischen Aufweitung des Stents nur Verformungen im Bereich von Fließgelenken konzentriert sind (so Beispielsweise in dem in Figur 1 gezeigten Stent 10). Die zweite Variante, bei der die Dilatation zu einer Verformung nahezu aller Strukturelemente 12 führt, ist exemplarisch in Figur 3 dargestellt. Es versteht sich von selbst, dass die Erfindung nicht auf die dargestellten Stentmuster beschränkt ist. Modifikationen des Stentdesign, die die Kontaktfläche vergrößern sind im allgemeinen bevorzugt, da bei wirkstoffbeladenen Beschichtungen eine gleichmäßigere Elution in die Gefäßwand ermöglicht wird. Ferner sind Bereiche hoher mechanischer Belastung, wie beispielsweise die Fließgelenke in Figur 1, entweder nicht zu beschichten oder es wird ein Stentdesign vorgegeben (z.B. das aus Figur 3), das die bei der Dilatation auftretenden Kräfte gleichmäßiger auf alle Strukturen des Stents verteilt. Damit soll eine Rissbildung oder ein Abblättern der Beschichtung infolge mechanischer Belastung vermieden werden.

Die Oberfläche 14 der Strukturelemente 12 ist mit einer polymeren Beschichtung 16 - hier angedeutet durch die dunkelschraffierte Fläche - bedeckt. Die polymere Beschichtung 16 erstreckt sich entweder über die gesamte Oberfläche 14 oder - wie hier dargestellt - nur über einen Teilabschnitt der Oberfläche 14. Die polymere Beschichtung 16 besteht aus Poly-L-lactid mit einem durchschnittlichen Molekulargewicht von > 200 kDa und weist Schichtdicken im Bereich von 3 bis 30 µm auf. Das Polymer ist biokompatibel und biodegradierbar. Ein Degradationsverhalten des Polymers kann durch Variation des Molekulargewichtes beeinflusst werden, wobei im allgemeinen eine Degradationszeit mit zunehmendem Molekulargewicht des Polymers ansteigt.

Die polymere Beschichtung 16 kann ferner als Träger für einen oder mehrere pharmakologisch aktive Wirkstoffe dienen, die über die Oberfläche 14 der Strukturelemente 12 an das umgebende Gewebe abgegeben werden sollen. Als Wirkstoffe kommen insbesondere Pharmaka aus der Gruppe Antikoagulatien, Fibrinolytika, Lipidsenker, Antianginosa, Antibiotika, Immunsupressiva, Zytostatika, PPAR-Agonisten, RXR-Agonisten oder einer Kombination derselben in Frage. So kann die polymere Beschichtung 16 insbesondere als Wirkstoff ein Fibrat oder eine Fibratkombination aus der Gruppe Clofibrat, Etofibrat, Etofyllinclofibrat, Bezafibrat, Fenofibrat und Gemfibrozil beinhalten. Auch Glitazone, wie Ciglitazon, Pioglitazon, Rosiglitazon und Troglitazon, sowie die RXR-Agonisten Bexaroten und Phytansäure sind aufgrund ihrer pharmakologischen Wirkung besonders geeignet. Die polymere Beschichtung 16 erlaubt eine kontrollierte Freisetzung der Wirkstoffe durch Diffusion bzw. allmähliche Degradation.

Da das Polymer biodegradierbar ist, kann die Elutionscharakteristik des Wirkstoffes durch Variation des Polymerisationsgrades beeinflusst werden. Mit steigendem Molekulargewicht des Polymers verlängert sich im allgemeinen auch der Zeitraum, in dem der Wirkstoff freigesetzt wird. Die Elutionscharakteristik einer derartigen polymeren Beschichtung wird vorzugsweise derart eingestellt, dass 10 bis 30 %, insbesondere 15 bis 25 %, des Wirkstoffes innerhalb der ersten zwei Tage freigesetzt wird. Der Rest des verbleibenden Wirkstoffes soll - ebenfalls gesteuert über Diffusions- und Degradationsvorgänge - sukzessive innerhalb der ersten Monate abgegeben werden.

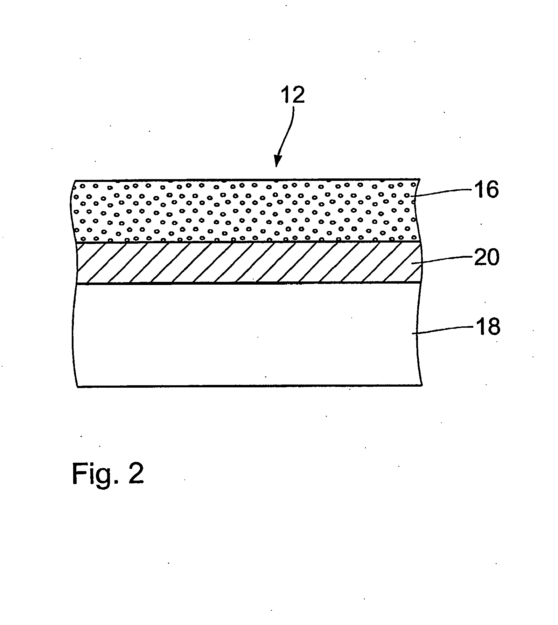

Eine besonders hohe Haftung auf der Oberfläche der Strukturelemente 12 kann dadurch erreicht werden, dass der Stent 10 an seiner Oberfläche 14 zusätzlich eine passive Beschichtung 20 aus amorphem Siliziumkarbid aufweist (siehe Figur 2). Die Herstellung derartiger Strukturen ist aus dem Stand der Technik, insbesondere aus dem Patent DE 44 29380 C1 der Anmelderin, bekannt und soll daher an dieser Stelle nicht näher erläutert werden. Es bleibt lediglich festzuhalten, dass ein Haftungsvermögen des polymeren Beschichtungsmaterials an der Stentoberfläche 14 mit einer solchen passiven Beschichtung 20 verbessert werden kann. Zudem mindert die passive Beschichtung 20 für sich allein bereits die neointimale Proliferation.

Eine weitere Verbesserung des Haftungsvermögens kann erzielt werden, wenn die Anbindung des Polymers auf kovalentem Wege mittels geeigneter Spacer oder durch Aufbringung einer Haftvermittlerschicht erfolgt. Grundzüge der Aktivierung der Siliziumkarbidoberfläche sind der DE 195 33682 A1 der Anmelderin zu entnehmen. Als Spacer können photoreaktive Substanzen wie Benzophenonderivate eingesetzt werden, die nach reduktiver Kopplung an die Substratoberfläche und gegebenenfalls Entschützung funktionelle Anbindungsstellen für das Polymer bereitstellen. Eine wenige Nanometer dicke Haftvermittlerschicht lässt sich beispielsweise durch Silanisierung mit Epoxyalkylalkoxysilanen oder Epoxyalkylhalogensilanen und deren Derivaten erzielen. Anschließend wird das Poly-L-Lactid durch Physisorpition bzw. Chemisorption an die Haftvermittlerschicht angebunden.

In der Figur 2 ist eine Schnittansicht durch ein Strukturelement 12 des Stents 10 in einem beliebigen Bereich desselben dargestellt. Auf einem Grundkörper 18 mit vorgenannter passiver Beschichtung 20 aus amorphem Siliziumkarbid ist die polymere Beschichtung 16 aufgebracht. Der Grundkörper 18 kann aus Metall oder einer Metalllegierung geformt sein. Soll der gesamte Stent 10 biodegradierbar sein, so kann der Grundkörper 18 insbesondere auf Basis eines biodegradierbaren Metalls oder einer biodegradierbaren Metalllegierung hergestellt werden. Besonders geeignet ist eine biodegradierbare Magnesiumlegierung. Auch derartige Materialien sind bereits ausreichend im Stand der Technik beschrieben worden, so dass auf eine gesonderte Darstellung verzichtet wird. Es wird in diesem Zusammenhang insbesondere auf die Offenbarung der DE 198 56983 A1 der Anmelderin verwiesen.

Die Herstellung der polymeren Beschichtung 16 wird mit Hilfe eines Rotationszerstäubers, der einen Nebel aus mikrofeinen Partikeln erzeugt, durchgeführt. Alternativ können auch Ultraschallzerstäuber eingesetzt werden. Die Beschichtung erfolgt schrittweise in zahlreichen Zyklen, die aus einem Benetzungsschritt des Stents im erzeugten Sprühnebel und einem anschließenden Trocknungsschritt des Niederschlags auf dem Stent durch Abblasen bestehen. Durch das mehrstufige Herstellungsverfahren lassen sich beliebige Schichtdicken und - sofern gewünscht - Konzentrationsgradienten des oder der Wirkstoffe in einzelnen Lagen der polymeren Beschichtung 16 erzeugen. Eine Sterilisation des Stents erfolgt durch Elektronenbeschuss, wobei ein teilweises Cracken der Polymerketten aufgrund des hohen Molekulargewichtes des Polymers in Kauf genommen werden kann. Die kinetische Energie der Elektronen liegt etwa im Bereich von 4 bis 5 MeV, da bei diesen Werten noch eine ausreichende Sterilisation bei nur geringer Eindringtiefe in den Grundkörper 18 des Stents 10 sichergestellt ist. Die Dosierung bewegt sich im Bereich von 15 bis 35 kGy pro Stent. Untersuchungen zeigten, dass keine oder nur eine minimale Minderung der biologischen Aktivität eingebetteter Wirkstoffe durch das Sterilisationsverfahren erfolgt.

Die erzeugten Schichtdicken der polymeren Beschichtung 16 liegen in der Regel im Bereich von 3 bis 30 µm. Besonders günstig sind Schichtdicken im Bereich von 8 bis 15 µm, da hier bereits eine weitestgehende Abdeckung der Oberfläche 14 des Stents 10 sichergestellt ist und noch nicht mit strukturellen Problemen wie Rissbildung und dergleichen gerechnet werden muss. Pro Stent 10 werden etwa 0,3 bis 2 mg, insbesondere 0,5 bis 1 mg, an Beschichtungsmaterial aufgebracht.

### Ausführungsbeispiel:

Ein handelsüblicher Stent, der unter dem Handelsnamen Lekton bei der Firma BI-OTRONIK beziehbar ist, wurde nachfolgend mit dem Polymer beschichtet.

Der Stent wurde in einen Rotationszerstäuber eingespannt. In einem Vorratsbehälter des Rotationszerstäubers wurde eine Lösung von Poly-L-lactid mit einem durchschnittlichen Molekulargewicht von 691 kDa in Chloroform bereitgestellt (Konzentration: 7,5g/l). Das Polymer kann als Granulat unter der Handelsbezeichnung Resomer© L 214 bei der Firma Boehringer Ingelheim bezogen werden. Als Wirkstoff wurde Clofibrat verwendet.

Der Stent wurde in je 80 Zyklen von jeweils ca. 10 s Dauer beidseitig mit einem durch den Rotationszerstäuber erzeugten fein verteilten Nebel benetzt. Der jeweiligen Benetzung folgte ein Trocknungsschritt durch Ablasen mit einer Dauer von ca. 12 Sekunden. Nach dem Ende von insgesamt 160 Beschichtungszyklen wird der Stent entnommen. Die Schichtdicke der polymeren Beschichtung liegt bei etwa 10 µm und die Masse der polymeren Beschichtung beträgt etwa 0,7 mg pro Stent.

Nach der Aufbringung der Beschichtung erfolgt eine Elektronenstrahlsterilisation des Stents mit 4,5 MeV-Elektronen bei einer Dosis von 25 kGy. Die Sterilisation mindert das durchschnittliche Molekulargewicht auf ca. 230 kDa (bestimmt nach Mark-Houwink).

Der implantationsfähige Stent wurde tierexperimentell am kardiovaskulären System des Schweins erprobt. Dazu wurde der Stent alternierend in den Ramus interventricularis anterior (RIVA), Ramus circumflexus (RCX) und die rechte Koronararterie (RCA) des Herzens von 7 Schweinen eingepflanzt. Zu Vergleichszwecken wurde zeitgleich ein Blindversuch mit Stents ohne Beschichtung gestartet. Nach 4 Wochen wurden die Restenoseraten der Stents mit und ohne polymere Beschichtung durch Ausmessung der neointimale Proliferation mittels quantitativer Koronarangiographie bestimmt und verglichen. Es ergab sich eine signifikante Senkung der neointimalen Proliferation bei der Verwendung eines Stents mit polymerer Beschichtung.

## Patentansprüche

1. Verfahren zur Herstellung eines implantationsfähigen Stents mit einer polymeren Beschichtung aus hochmolekularem Poly-L-lactid, **dadurch gekennzeichnet, dass** der Stent (10)
(a) zumindest abschnittsweise mit einem feinen Nebel aus einer Lösung von Poly-L-lactid des durchschnittlichen Molekulargewichts von mehr als 650 kDa benetzt wird,
(b) die auf dem Stent (10) aufgebrachte Lösung durch Abblasen getrocknet wird und
(c) der Stent (10) anschließend mittels Elektronenstrahlsterilisation mit einer Dosierung im Bereich von 15 bis 35kGy und einer kinetischen Energie der Elektronen im Bereich von 4 bis 5 MeV sterilisiert wird.

2. Verfahren nach Anspruch 1, **dadurch gekennzeichnet, dass** die Verfahrensschritte Benetzen und Abblasen so oft wiederholt werden bis die polymere Beschichtung (16) eine Schichtdicke von 3 bis 30 µm aufweist.

3. Verfahren nach Anspruch 1, **dadurch gekennzeichnet, dass** die Elektronenstrahlsterilisation mit einer Dosierung im Bereich von 22 bis 28 kGy durchgeführt wird.

## Claims

1. A method for manufacturing an implantable stent having a polymer coating made of high-molecular-weight poly-L-lactide, **characterized in that** the stent (10)
(a) is at least partially wetted using a fine mist made of a solution of poly-L-lactide of an average molecular weight of more than 650 kDa,
(b) the solution applied to the stent is dried through blowing, and
(c) the stent is subsequently sterilized using electron beam sterilization having a dosing in the range from 15 to 35 kGy and a kinetic energy of the electrons in the range from 4 to 5 MeV.

2. The method according to Claim 1, **characterized in that** the method steps of wetting and blowing are repeated until the polymer coating (16) has a layer thickness of 3 to 30 µm.

3. The method according to Claim 1, **characterized in that** the electron beam sterilization is performed using a dosing in the range from 22 to 28 kGy.

## Revendications

1. Procédé pour la fabrication d'un stent apte à l'implantation avec un revêtement polymère à base de poly-L-lactide à poids moléculaire élevé, **caractérisé en ce que** le stent (10)
(a) est humecté au moins par envoi avec un brouillard fin à base d'une solution de poly-L-lactide du poids moléculaire moyen de plus de 650 kDa,
(b) la solution appliquée sur le stent (10) est séchée par soufflage et
(c) le stent est stérilisé ensuite au moyen d'une stérilisation par faisceau électronique avec un dosage de l'ordre de 15 à 35 kGy et une énergie cinétique des électrons dans la plage de 4 à 5 MeV.

2. Procédé selon la revendication 1, **caractérisé en ce que** les étapes du procédé (humectation et soufflage) sont répétées jusqu'à ce que le revêtement (16) polymère présente une épaisseur de couche de 3 à 30 µm.

3. Procédé selon la revendication 1, **caractérisé en ce que** la stérilisation par faisceau électronique est effectuée avec un dosage dans la plage de 22 à 28 kGy.
